# EUROPÄISCHE PATENTSCHRIFT

(11) **EP 0 731 687 B1**
(45) Veröffentlichungstag und Bekanntmachung des Hinweises auf die Patenterteilung: **07.03.2001**
(21) Anmeldenummer: 95902709.5
(22) Anmeldetag: 12.12.1994
(51) Int. Cl.: A61K 7/50

(54) **REINIGUNGS- UND/ODER PFLEGEMITTEL FÜR HAARE UND VERFAHREN ZU DEREN HERSTELLUNG**
HAIR CLEANING AND/OR CARE AGENT AND PROCESS FOR PRODUCING IT
PRODUITS DE LAVAGE ET/OU DE SOINS POUR LES CHEVEUX, ET LEUR PROCEDE DE FABRICATION

(30) Priorität: 10.12.1993 AT 250193
(43) Veröffentlichungstag der Anmeldung: 18.09.1996
(73) Patentinhaber: Petritsch, Erich, 1090 Wien (AT)
(72) Erfinder: Petritsch, Erich, 1090 Wien (AT)
(74) Vertreter: Brauneiss, Leo, Dipl.Ing.
(86) Internationale Anmeldenummer: AT9400194
(87) Internationale Veröffentlichungsnummer: WO9515745

(56) Entgegenhaltungen:
- EP-A- 0 150 250
- EP-A- 0 498 272
- WO-A-89/03670
- PATENT ABSTRACTS OF JAPAN vol. 11, no. 163 (C-424) 26. Mai 1987 & JP,A,61 293 908 (KAO CORPORATION) 24. Dezember 1986
- PATENT ABSTRACTS OF JAPAN vol. 12, no. 191 (C-501) 3. Juni 1988 & JP,A,62 294 604 (MITSUI TOATSU CHEM INC) 22. Dezember 1987
- DIE KOSMETISCHEN PRäPARATE., AUGSBURG Seiten 672 - 674 G. A. NOWAK 'Badesalze und Badetabletten'

## Beschreibung

Die Erfindung betrifft den aus dem modernen Leben nicht mehr wegzudenkenden, vom den persönlichen Bedürfnissen nach Gesundheit, Hygiene und Ästhetik bis weit in den human-sozial-ökonomischen, dienstleistungsgewerblichen und kosmetikindustriellen Sektor hinein sich erstreckenden Bereich der Reinigungs- und/oder Pflegemittel, im speziellen für Haare.

Als wesentlicher Bestandteil des modernen Lebens ist weiters sowohl im privaten wie im gewerblichen Bereich der Faktor der Umweltverträglichkeit und zwar sowohl, was die Inhaltsstoffe der genannten Mittel, deren Grenzen jedoch durch die Notwendigkeit einer Humanverträglichkeit wenigstens einigermaßen abgesteckt sind, als auch ganz besonders deren Umhüllung, Verpackung, Präsentation, Umverpackung und deren Entsorgung betrifft, von ganz wesentlicher und immer noch steigender Bedeutung.

Seit geraumer Zeit behaupten auf dem Sektor der Haar-Reinigungs- und -Pflegemittel mit in vielen Fällen auch ineinander übergehender Funktionalität die unterschiedlichsten, aber immer in dickflüssiger, flüssig- cremiger oder cremiger Form vorliegenden Shampoons mit den verschiedensten Detailaufgaben in praktisch unangefochtener Weise das gesamte Feld dieses Sektors der Keralogie.

In besonders handhabungsfreundlicher, jedoch umweltbedenklicher Weise ist, beginnend vom in Einzelportionen für jeweils einen Haarwasch- oder Pflegevorgang, insbesondere in Briefchen, Polstern, Beuteln, Blistern oder Säckchen abgepackten Shampoons bzw. Pflegemitteln für Haare bis hin zum wettbewerbsbedingt äußerst werbewirksamen, designmäßig aufwendig durchgestylten Behälter bzw. einer derartigen Flasche mit Spezialverschluß, und alles möglichst in Form von aufbauend in sich geschlossenen Haar-Pflege-Sets oder -Serien, alles vertreten, was sich an Variationen auf dem Gebiet der Verpackungstechnik anbietet. Abgesehen von Glasgebinden sind es die verschiedensten Arten von Kunststoffen, welche dieses Gebiet beherrschen, über deren Entsorgungsproblematik, insbesondere infolge ihres ja im tagtäglichen Gebrauch anfallenden Mengen-Ausmaßes hier keine näheren Ausführungen notwendig scheinen.

Die Erfindung hat sich angesichts der an sich nicht besonders auffallenden, jedoch tatsächlich absolut nicht zu unterschätzenden Dimension des aufgezeigten Problems die Aufgabe gestellt, auf dem Haarpflegesektor in möglichst umweltgerechter Weise eine wesentliche Reduzierung und Minimierung der einschlägigen Verpackungs-Mengen und insbesondere auch -Volumina zu erreichen, ohne dadurch die Handhabungsfreundlichkeit dieser Haarpflegemittel nur irgendwie herabzusetzen.

Es wurde gefunden, daß unter Vermeidung der unangenehmen Handhabung von Pulvern auf dem Haarpflegesektor zur Erreichung dieser Ziele an sich eine kompakte feste Form von Shampoos und Haarpflegemitteln große Vorteile brächte, jedoch entspricht die Geschwindigkeit der Verteilung und Auflösung von deren wirksamen Substanzen bei der Reinigungs- und/oder Pflegebehandlung der Haare keineswegs den gängigen Bedürfnissen des modernen Konsumenten nach "Instant"-Bereitstellung. Es war also eine Gesamtkonzeption einer Art Haar-Shampoo oder -Kur-Tablette zu entwickeln, welche aber ein - eine mit der Verteilung eines üblichen Shampoons im nassen Haar durchaus vergleichbare rasche Zerteilung - förderndes System aufweisen muß.

Zum allgemeinen Stand der Technik der Verteilung von Stoffen mit Hilfe von Gasen ist folgendes zu bemerken:

In der AT-PS 253 123 wird ein geformter fester Badezusatz beschrieben, welcher keinen Bezug auf eine im Vergleich zu einem Voll-Körperbad völlig anders verlaufende "Haarwäsche" nimmt. Dieser Badezusatz, welcher kein Haarreinigungsmittel darstellt, ist so gestaltet, daß nicht erst in situ eine Gasentwicklung bei Kontakt des Mittels mit dem Reinigungswasser angestrebt wird, vielmehr hat dort die CO₂-Entwicklung schon bei der Herstellung des Badezusatz-Formkörpers stattgefunden und hat noch vor dessen Fertigstellung schon völlig aufgehört. Das Gas dient dort nur zur Schaumentwicklung und ist in Porenform schon fertig in der Tensidschmelze eingeschmolzen. Es ist zu einem "Zerfallenlassen" bzw. mechanischen Zersetzen der Tablette und zur Verteilung von deren Tensid durch eine spontane Gasentwicklung infolge einer situ-Expansion nicht fähig.

Eine Anregung oder auch nur Andeutung in Richtung auf ein spezifisch für Haarreinigung vorgesehenes Pflegemittel enthält diese Patentschrift nicht.

Der EP-A2 330 435 läßt sich als festes Kompakt-Haarwaschmittel nur eine etwas modifizierte Seife in üblicher Form und üblicher Art ohne jedes Verteilungssystem für dieselbe entnehmen, welche die bei der Haarwäsche vom Konsumenten erwartete Sofort-Bereitstellung der waschaktiven Tenside nicht sicherstellen kann.

Die EP-A1 150 250 befaßt sich ganz allgemein mit der Verteilung von Wirkstoffen, wie z.B. ätherischen Ölen, Parfum, therapeutischen Wirkstoffen mit geringen Einsatzkonzentrationen in größeren Wassermengen und schlägt verschiedene Formkörper-Arten - allerdings nur für das Gasemittier-System selbst, jedoch ohne Hinweis und Rücksicht auf die zu verteilenden Stoffe, und deren Mengen, die im Falle von Tensiden und Seifen in hohen Anteilen vorliegen - vor.Gerade deren Eigenschaften können entscheidend sein für die Effektivität eines solchen Verteilsystems. Wenn die kompaktierten Komponenten "schmierenden" Charakter haben, wie z.B. eben bei Seifen und Tensiden der Fall, so tritt eine "Blockierung" des Zutritts des Wassers zum CO₂-Entwicklungssystem ein.

Darüber hinaus sind Bad-Zusätze mit einem "Gassprudelsystem" gemäß der genannten DE-A1 für Vollbäder auch deswegen für die Haarwäsche-Problematik nicht relevant, weil in Vollbädern unverhältnismäßig große Mengen des Lösungsmittels Wasser zur Verfügung stehen und auch mehr Zeit.

Bei einer Haarwäsche soll ein Haarreinigungs- und Pflegemittel (wie flüssiges Shampoo) in die hohle feuchte Hand genommen und dann unter Bewegung beider Hände im genäßten Haar verteilt werden, wobei aber eine sofortige Schaumbildung erwartet wird, was bei einem Bad bzw. Schaumbad keineswegs so kritisch ist, wobei dort ungleich mehr Wasser zur Verfügung steht und dieses sprudelnd in eine Wanne eingelassen wird.

Weiters ist der US-A1 3 328 307 eine Bad-Zubereitung mit speziellen Tensiden auf Basis von quaternären Ammoniumverbindungen mit Carboxylgruppen zu entnehmen, wobei dort der Schwerpunkt auf flüssigen Zubereitungen liegt. Allgemein ist auch die Möglichkeit von festen Formen erwähnt, welche durch Vermischen der dortigen Tenside mit Trägerstoffen oder Verdickungsmitteln erhältlich sind. Im Beispiel III ist dort ein Vermischen einer durch Aufkonzentrierung einer flüssigen Tensidzubereitung erhaltenen Feststoff-Mischung mit Natriumchlorid, Natriumbicarbonat und Weinsäure sowie Pinienöl und Fluorescein beschrieben, wobei die so erhaltene Masse in Tablettenform kompaktiert wird.

Was die angestrebte Verteilung der Tenside betrifft, wird dazu in dem Beispiel besonders betont, daß die Tabletten in einem stark strömenden Wasserstrahl positioniert werden müssen, also mit viel und besonders stark bewegtem Wasser in Berührung gebracht werden müssen. Damit konnte auch diese US-A1 zur Frage einer Entwicklung eines neuen in Feststoff-Form vorliegenden Haarwaschmittels nichts beitragen.

Die Schaumbad-Tabletten gemäß WO-A 89 03 670 sind bezüglich ihrer Zusammensetzung ganz gezielt und ausschließlich auf ihren Einsatz für die Bereitung von sprudelnden Schaumbädern abgestimmt und es sind dort keinerlei Hinweise oder Andeutungen bezüglich anderer Verwendungszwecke enthalten. Aus der großen Zahl der in Frage kommenden Tenside und CO₂-Entwicklungssysteme beschränkt sich dort die Auswahl auf vier ganz genau spzifizierte, materialmäßig nicht variierbare schäumungs- und schaumhalte-aktive Substanzen, was nur so zu interpretieren ist, daß jede der in der WO-A gemachten Angaben bezüglich Substanzen und deren Mengenverhältnisse zueinander ausschließlich auf die Optimierung von Schaum-Sprudel-Bädern ausgerichtet ist.

Dem Pat. Abstr. Jap. JP 62 294 604 ist ein Haar-Detergent, welches fest ist, zu entnehmen, dort wird nur eine feste Substanz erwähnt, von deren Ausgestaltung als Tablette ist an keiner Stelle die Rede, und eine Tablettenform wird von dieser Schrift auch nicht implizit angeregt. Es sei dazu nur darauf verwiesen, daß z.B. Pulver feste Substanzen sind.

Es ist in diesem Abstract für das CO₂-Entwicklungssystem des dortigen Haar-Reinigungsmittels ein Mengenbereich genannt und auch die dort vorgesehene, wasserlösliche, hochpolymere Substanz ist mengenmäßig definiert. Es fehlen jedoch jegliche Mengen-Angaben bezüglich der für ein Haar-Reinigungsmittel absolut wichtigen Tenside, hingegen sind gemäß diesem Abstract andere Komponenten, wie Ingwer und ein wasserlösliches Polymer, zwingend vorgesehen.

Zum Pat. Abstr. Jap. JP 61 293 908 ist festzuhalten, daß bei dem dort geoffenbarten Reiniger das angenehme Gefühl bei dessen Verwendung und die Anregung des Blutkreislaufs durch die Wirkung des sich beim Kontakt mit Wasser entwickelnden CO₂-Gases im Vordergrund stehen. Dies weist massiv darauf hin, daß der aus diesem Abstract bekannte Reiniger seinen Schwerpunkt in der Reinigung und Pflege der Haut hat und gezielt auf diesen Anwendungszweck gerichtet ist und daß die Erwähnung der Haareinigung eine absolut untergeordnete Funktion betrifft. Einen wesentlichen Bestandteil des Reinigers gemäß dem Abstract stellt die neben dem dort enthaltenen Schäumsystem und dem Tensid die Gegenwart von Zeolithen dar. So soll die dort geoffenbarte Zusammensetzung neben 0,5 bis 35 Gew.% des von einem Carbonat und einer Säure gebildeten Schäumsystems 1 - 40 Gew.% eines Zeolithen enthalten. Im gesamten Abstract ist jedoch über dessen Gehalt an Tensiden keinerlei Angabe enthalten. Dieser Gehalt kann bei der Annahme, daß der Reiniger 100 Gew.% Komponenten insgesamt aufweisen müßte, theoretisch leicht errechnet werden. Es können demnach minimal 25 Gew.% und maximal 98,5 Gew.% festes Tensid im Reiniger mit der im Abstract beschriebenen Zusammensetzung enthalten sein.

Aus der täglichen Lebenserfahrung wird klar, daß ein festes Tensid, das in überwiegenden Mengen von z.B. bis zu 98,5 Gew.% neben nur geringfügigen Mengen an CO₂-Entwicklungssystem und Zeolith in einem Reiniger enthalten ist, das also z.B. nur 1 Gew.% an einem CO₂-Entwicklungssystem beinhaltet, kaum zu einem auch nur einigermaßen brauchbar schäumenden Bad zu führen imstande wäre. Der exorbitante Überschuß an Tensid würde die Schäumwirkung in der Praxis völlig unterdrücken, die bei einer Haarwäsche ganz wichtige, rasche Desintegration einer derartigen Tablette ist demnach nicht gegeben. Möglicherweise könnte das im genannten Abstract als im dort beschriebenen Reiniger zwingend vorgesehene und genau spezifizierte Zeolith-Material ein Mittel sein, diesem Problem abzuhelfen. Dafür spricht, daß gemäß diesem Abstract ein offenbar wichtiges bevorzugtes Merkmal darin besteht, daß an dem zwingend in der festen Reinigungszusammensetzung enthaltenen Zeolith-Material ein Gas, insbesondere CO₂, direkt adsorbiert ist. Dadurch könnte dem ganz offenkundigen Mangel an CO₂-Entwicklungskraft für ein Schäumen nachgeholfen werden.

Gegenstand der Erfindung ist ein neues Reinigungs- und Pflegemittel für Humanhaar in Form von kompaktierten Formkörpern oder Tabletten auf Basis eines festen Gemenges bzw. Gemisches mit mindestens einem haar- und hautverträglichen, in Festphase vorliegenden Tensid mindestens einem Zusatz - und/oder Hilfsstoff und einem bei Kontakt mit Wasser CO₂ oder ein anderes physiologisch unbedenkliches Gas freisetzenden, bevorzugt mit einer Kombination von mindestens einem Carbonat und/oder Carbaminat, und/oder Hydrogencarbonat und mindestens einer in fester Phase vorliegenden organischen Säure gebildeten Grundkörper, dadurch gekennzeichnet, daß es in Form von Reinigungs- und Pflege-Einheiten oder -Subeinheiten mit einem Volumen von jeweils 0,5 bis 2,5 cm³, insbesondere von 1 bis 1,5 cm³, bezogen auf die Gesamtmasse des Formkörpers bzw. der Tablette, folgende Grundzusammensetzung aufweist:

| | | |
|---|---|---|
| a) | gasgenerierendes Grundkörper-System: | 40-60% |
| b) | Tensid(e): | 25-45% |
| c) | haar- und kopfhautpflegesame Wirkstoff(e): | 3-12% |
| d) | Hilfsstoff(e) und Additiv(e) und Magnesiumoxid und/oder Alkalialuminat als Stabilisatoren | 1- 5%, |

wobei es aus den beiden letztgenannten Stoffgruppen c) und d) mindestens einen - an sich bekannten - haar- und hautpflegewirksamen Wirkstoff aus den Gruppen der
hautverträglichkeits-fördernden Wirkstoffe und Anti-Irritantien, vorzugsweise Allantoin,
Radikalfänger, vorzugsweise Harnstoff,
Avivagewirkstoffe, vorzugsweise Guarhydroxypropyltrimethylammoniumchlorid, Wuchsfördermittel, vorzugsweise Propandiol,
Schuppenwirkstoffe, vorzugsweise Octopirox, Pyrithionsalze, Selendisulfid, Antifettwirkstoffe, vorzugsweise eine Kombination verschiedener (Thio-)Aminosäuren oder Isopropylmyristat,
Fettungshemmstoffe, vorzugsweise Aminodermin oder Eiweißhydrolysate, sowie der Finalpflegestoffe, vorzugsweise Seidenproteine, Kräuter-Trockenextrakte und/oder ätherische Öle bzw. deren Gemische,
mindestens einen Stabilisator aus der Gruppe Magnesiumoxid und Alkalialuminate und mindestens einen Hilfsstoff bzw. mindestens ein Additiv aus den Gruppen der Kompaktier- bzw. Preßhilfsmittel, vorzugsweise Talkum,
Füllmittel, vorzugsweise Natrium- oder Magnesiumsulfate,
Sprengmittel, vorzugsweise Stärke, Cellulosederivate, Carboxymethylcellulose, Alginate, Siliciumdioxide oder Titandioxide,
Gleitmittel bzw. Glanzmittel, vorzugsweise gehärtetes Rhizinusöl, Metallseifen u.dgl., Trockenbindemittel, vorzugsweise Saccharose, Lactose oder Sorbit,
sowie der
Färbemittel bzw. Farbstoffe
enthält, und wobei die Säure im gasgenerierenden Desintegrations-Grundkörper in Relation zu dessen Gasentwicklungskomponente in stöchiometrisch molarem Überschuß vorliegt und das Reinigungs- und Pflegemittel nach Desintegration, Verteilung und Auflösung des Formkörpers bzw. der Tablette in der wässerigen Phase einen Gesamt-pH-Wert von unter 6,8, insbesondere von 3-6, aufweist.

An dieser Stelle soll nicht unerwähnt bleiben, daß bei dem neuen Mittel selbstverständlich eine pflegende Reinigung haarunmittelbarer und haarnaher Bereiche nicht auszuschließen ist.

Durch den Einbau des auf Festphasebasis beruhenden, selbsttätigen und erst bei Wasserkontakt effektuierten Desintegrations- und Schäumungs-Systems im oben angeführten Mengen-Verhältnisbereich zu dem Tensidanteil wurde erreicht, daß die Tablette und insbesondere deren Inhaltsstoffe sofort, beginnend vom ersten Kontakt mit dem nassen Haar oder gegebenenfalls sogar schon in der feuchten Hand im Verein mit den üblichen reibenden Waschbewegungen der Hände, also durch den manipulativen Kontakt, in äußerst kurzer Zeit über den zu reinigenden Haarbereich verteilt wird und ihre reinigungsaktive und -effektive Wirkung entfaltet bzw. entfalten.

Dieser Mechanismus der zeitminimierten und gleichmäßigen Verteilung hat den Effekt, daß infolge der Frischgasentwicklung eine individuelle, als angenehm empfundene Art Mineralwasser-Erfrischungseffekt zu beobachten ist.

Durch die neue Form der Tabletten ist weiters in umweltfreundlicher Weise dafür Sorge getragen, daß eine flüssigkeitsdichte, druckfeste Verpackung wegfällt und sogar ein portionsweises Angebot, z.B. 1 Tablette für eine Haar-Vorwäsche und 1 Tablette für die Haar-Hauptwäsche u.dgl. nur mehr einer einfachen Gesamtverpackung, z.B. auf Kartonbasis, für alle Tabletten bedarf, die im günstigen Fall auch durch einen recyclingfähigen, oftmalig wiederbefüllbaren, werbewirksam und ästhetisch anspruchsvollen, formschönen Glasbehälter od.dgl. gebildet sein kann. Beim Gebrauch einer Tablette fallen selbstverständlich zusätzlich alle Unannehmlichkeiten weg, die beim Öffnen von flüssigen Inhalt aufweisenden Portioniersäckchen mit Shampoo auftreten, wie Aufplatzen oder Nichtauffindung eines Stech- oder Schneidwerkzeuges zum Öffnen der Verpackungsfolie u.dgl.

Die wesentlichen Bestandteile der neuen Shampoo-Tabletten sind durch das Gasgenerativ-System und die Tenside im allgemeinen gegeben und es wird durch die oben angeführten begleitenden Wirkstoffe auch auf die Pflege besondere Rücksicht genommen. Dementsprechend ist der Einbau der oben zusammengefaßten Wirkstoffklassen und individuell als bezurzugend genannten Wirkstoffe in die neuen Haarwasch-Tabletten von besonderem Vorteil.

Zu den bezüglich Pflege, Glanz und Geruchsindividualität besonders bevorzugten ätherischen Ölen in Tabletten für den gehobenen Bedarf, z.B. im Salon, als Pflegeserie usw., seien nur beispielhaft Ylang-Ylang, Sandelholz, Zedernholz, Rosenholz, Bergamotte, Lavendel, Melisse, Geranium, Patchouli, Wacholder, Pfefferminze, Rosmarin, Orange, Zitrone, Fenchel und Mischungen von drei oder vier der genannten Öle angeführt.

Kräuter-Trockenextrakte, z.B. CO₂-Extrakte, sind eher für den üblichen Bedarf im Haushalt vorgesehen.

Insbesondere tensidbezogen sind die auf die Tablettierung und die Stabilität der neuen Tabletten bezogenen oben angeführten Hilfsstoffe bzw. Additive. Deren im Erfindungszusammenhang besonders bevorzugten Klassen ind Individuen sind oben ebenfalls genannt, wobei den die Desintegration und damit schnelle und gleichmäßige Gasgenerierung fördernden Sprengmitteln selbstverständlich besondere Bedeutung zukommt.

Bei dem im CO₂-Entwicklungssystem der Haarwaschtabletten vorgesehenen stöchiometrischen Überschuß der Feststoff-Säure-Komponente im schaumgasgenerierenden Grundkörper ist, wie gefunden wurde, ein Optimum an Gleichmäßigkeit und Effektivität der Naßverteilung der neuen Haarwasch- und -pflegemittel im Haar unter den praktischen, im Haushalt oder im Friseurbetrieb gegebenen und vom Konsumenten erwarteten Bedingungen sichergestellt.

Im Zusammenhang damit, aber für die Schonung des vom Waschvorgang chemisch und mechanisch beanspruchten Haares, steht das weitere Merkmal, das neue Festphase-Haar-Reinigungsmittel innerhalb des oben angegebenen effektiven pH-Wertbereiches arbeiten zu lassen. Dabei ist es für die neuen Haarreinigungsmittel von Vorteil, wenn sie zumindest in den schwachsauren Bereich hineinreichend angelegt sind.

Was die Zusammensetzung der neuen Festphase-Haarwaschmittel betrifft, haben sich Formkörper bzw. Shampootabletten mit Gehalten der wesentlichen, sie aufbauenden Komponenten gemäß **Anspruch** 2 besonders bewährt.

Die derart zusammengesetzten, neuartigen Haar-Waschtabletten zeichnen sich durch besonders rasche Desintegrierbarkeit beim Kontakt mit Wasser in flüssiger Form aus, sie sind aber selbst gegen länger andauernde, hohe Luftfeuchtigkeit, wie sie z.B. in Sanitärräumen und Badezimmern vorherrscht, durchaus resistent und neigen nicht zum Zusammenbacken.

Soll neben dem Reinigungseffekt das neue Kompakt-Mittel auch Vorteile für eine Haar- und eine dabei nicht auszuschließende Kopfhaut-Pflege haben, bieten sich als Tenside ganz besonders kationische Tenside aus den vom **Anspruch** 3 umfaßten Gruppen an.

Auch hier bringen innerhalb dieser Gruppe aufgrund einschlägiger Erfahrungen, insbesondere im Zusammenhang mit dem chemisch-basiert schaumgas-generierenden, Grundkörper-System die aus **Anspruch** 4 hervorgehenden individuellen, kationischen Tenside besondere Vorteile.

An sich haben die Haarkur- bzw. Haarpflegezusätze im neuen Reiniger die Aufgabe, neben der Haarreinigung die Geschmeidigkeit, Frisierbarkeit, die Fülle, den Griff und den Glanz des Haares zu erhöhen und diese Eigenschaften zwischen den Waschgängen zu erhalten. Dabei kommt insbesondere der Schließung der Schuppenschicht der Haare selbst durch adäquaten pH-Wert und Direktanlagerung von Nährstoffen besonderer Vorrang zu, es wird auf diese Weise eine Art Schutzschicht auf dem Haar gebildet.

Porosität der neuen Haar-Reinigungstabletten gemäß **Anspruch** 5 hat den Vorteil, eine noch mehr erleichterte Desintegrierbarkeit derselben herbeizuführen, da bei Kontakt mit nassem Haar u.dgl. infolge Kapillarwirkung das zur Schäumung bzw. Gasfreisetzung aus dem Grundkörper notwendige Wasser sofort auch in das Innere der Tablette vordringt und dort noch schneller seine gasentwickelnde Wirkung ausüben kann.

Zur konsumenten- und gewerbefreundlichen Vermeidung von Verwechslungen und im Sinne des Gedankens der Schaffung von Wasch- und Pflege-Serien kann in besonders günstiger Weise auch eine zweckgerichtete Farbgebung gemäß **Anspruch** 6 vorgesehen sein.

Vom Standpunkt der Haar- und Hautverträglichkeit und ihrer Verteilungsrate besonders vorteilhaft sind Haar-Reinigungsmittel, deren desintegrationsförderndes, gasgenerierendes System mit den im **Anspruch** 7 genannten Komponenten gebildet ist.

Zum im Anspruch 7 aufscheinenden Begriff "Pseudoalkali" seien Ammonium und einfach - oder mehrfach alkyliertes Ammonium angeführt. Der oben in Klammern aufscheinende Ausdruck "(poly-)" in den chemischen Verbindungen bedeutet "mono-, di- oder poly-".

Eine die Wirkstoff-und Tensidverteilung beim Wasch- und Pflegevorgang besonders effektiv fördernde, gleichmäßige Gasentwicklung und Gasbläschen geringer Dimension generierende Kombination der Komponenten des Grundkörpers bieten in vorteilhafter Weise Shampootabletten gemäß **Anspruch** 8.

Im besonderen ist bezüglich Tensid einer Zusammensetzung der neuen Formkörper bzw. Tabletten gemäß **Anspruch** 9 der Vorzug zu geben.

Von den im Anspruch 9 genannten Klassen anionischer Tenside ist den aus dem **Anspruch** 10 hervorgehenden, individuellen Tensiden hinsichtlich ihrer curativen, längerzeitigen Wirkung über den Waschvorgang selbst hinaus ein besonderer Vorzug zu geben.

Angenehm im Gebrauch, einen Schutz vor Nässe bietend und umweltschonend ist die gemäß **Anspruch** 11 vorgesehene Unterbringung der neuen Haar-Reinigungstabletten in einem Dispenser od.dgl.

Was die Herstellung des neuen Kompakt- bzw. Festphase-Shampoos betrifft, so unterscheiden sich diese nicht wesentlich von üblichen Tablettierverfahren, bei denen feinteilige Feststoffgemische kompaktiert werden.

Ist jedoch einer der Komponenten an sich flüssig, so ist es fertigungstechnisch von besonderem Vorteil, gemäß **Anspruch** 12 zu verfahren, da hiebei eine Art Aufsaugeffekt genutzt wird und auf diese Weise dann nur Feststoffe zu Tabletten zu verarbeiten sind.

### Beispiel:

Es wurde ein Ansatz von 1,8 kg einer Ausgangsmischung für ein Haarwaschmittel in Tablettenform mit folgenden Komponenten in der folgenden Zusammensetzung hergestellt:

| | |
|---|---|
| 5 % | Talkum (DAB), worin |
| 0,8 % | Zedernholzöl aufgesaugt waren, |
| 56 % | eines 1:1,1-Mol-Gemisches von Natriumhydrogencarbonat und Citronensäure (alles DAB) |
| 33,6 % | Laurylsulfonsäureester-Na-Salz |
| 0,2 % | Allantoin |
| 0,5 % | Octopirox^{R} |
| 1,2 % | Guarhydroxypropyltrimethylammoniumchlorid und |
| 2 % | Maisstärke |

Die Tabletten hatten 1,3 cm Durchmesser und 0,75 cm Höhe.

Die erhaltenen Haarwaschtabletten zeigten bei ihrer Verwendung gleich auf der feuchten Hand Auflösungstendenzen unter sofort einsetzender CO₂Entwicklung und während des Einbringens bzw. Einmassierens der Tablette in das Haar führt dieser durch die Gasentwicklung geförderte Vorgang die völlige Auflösung der Tabletteninhaltsstoffe innerhalb von etwa 20 bis 25 s herbei. Die Reaktion der entstehenden Waschlösung lag im neutral-sauren pH-Bereich bei etwa 6,5.

Das Haar wies nach Trocknen und Kämmen hohen Glanz bei festem Griff und ausgesprochen angenehmer Kämmbarkeit auf. Da die Shampootabletten aus einer größeren weiter verwendbaren Packung (Glas) entnommen wurden, fiel keinerlei Abfall an.

## Patentansprüche

1. Reinigungs- und Pflegemittel für Humanhaar in Form von kompaktierten Formkörpern oder Tabletten auf Basis eines festen Gemenges bzw. Gemisches mit mindestens einem haar- und hautverträglichen, in Festphase vorliegenden Tensid mindestens einem Zusatz - und/oder Hilfsstoff und einem bei Kontakt mit Wasser CO₂ oder ein anderes physiologisch unbedenkliches Gas freisetzenden, bevorzugt mit einer Kombination von mindestens einem Carbonat und/oder Carbaminat, und/oder Hydrogencarbonat und mindestens einer in fester Phase vorliegenden organischen Säure gebildeten Grundkörper, dadurch gekennzeichnet, daß es in Form von Reinigungs- und Pflege-Einheiten oder -Subeinheiten mit einem Volumen von jeweils 0,5 bis 2,5 cm³, insbesondere von 1 bis 1,5 cm³, bezogen auf die Gesamtmasse des Formkörpers bzw. der Tablette, folgende Grundzusammensetzung aufweist:
| | | |
|---|---|---|
| a) | gasgenerierendes Grundkörper-System: | 40-60% |
| b) | Tensid(e): | 25-45% |
| c) | haar- und kopfhautpflegesame Wirkstoff(e): | 3-12% |
| d) | Hilfsstoff(e) und Additiv(e) und Magnesiumoxid und/oder Alkalialuminat als Stabilisatoren | 1- 5%, |
wobei es aus den beiden letztgenannten Stoffgruppen c) und d) mindestens einen - an sich bekannten - haar- und hautpflegewirksamen Wirkstoff aus den Gruppen der
hautverträglichkeits-fördernden Wirkstoffe und Anti-Irritantien, vorzugsweise Allantoin, Radikalfänger, vorzugsweise Harnstoff,
Avivagewirkstoffe, vorzugsweise Guarhydroxypropyltrimethylammoniumchlorid,
Wuchsfördermittel, vorzugsweise Propandiol,
Schuppenwirkstoffe, vorzugsweise Octopirox, Pyrithionsalze, Selendisulfid,
Antifettwirkstoffe, vorzugsweise eine Kombination verschiedener (Thio-)Aminosäuren oder Isopropylmyristat,
Fettungshemmstoffe, vorzugsweise Aminodermin oder Eiweißhydrolysate, sowie der Finalpflegestoffe, vorzugsweise Seidenproteine, Kräuter-Trockenextrakte und/oder ätherische Öle bzw. deren Gemische,
mindestens einen Stabilisator aus der Gruppe Magnesiumoxid und Alkalialuminate und mindestens einen Hilfsstoff bzw. mindestens ein Additiv aus den Gruppen der
Kompaktier- bzw. Preßhilfsmittel, vorzugsweise Talkum,
Füllmittel, vorzugsweise Natrium- oder Magnesiumsulfate,
Sprengmittel, vorzugsweise Stärke, Cellulosederivate, Carboxymethylcellulose, Alginate, Siliciumdioxide oder Titandioxide,
Gleitmittel bzw. Glanzmittel, vorzugsweise gehärtetes Rhizinusöl, Metallseifen u.dgl., Trockenbindemittel, vorzugsweise Saccharose, Lactose oder Sorbit,
sowie der
Färbemittel bzw. Farbstoffe
enthält, und wobei die Säure im gasgenerierenden Desintegrations-Grundkörper in Relation zu dessen Gasentwicklungskomponente in stöchiometrisch molarem Überschuß vorliegt und das Reinigungs- und Pflegemittel nach Desintegration, Verteilung und Auflösung des Formkörpers bzw. der Tablette in der wässerigen Phase einen Gesamt-pH-Wert von unter 6,8, insbesondere von 3-6, aufweist.

2. Reinigungs- und Pflegemittel nach Anspruch 1, dadurch gekennzeichnet, daß es, bezogen auf die Gesamtmasse des Formkörpers bzw. der Tablette, folgende Grundzusammensetzung aufweist:
| | |
|---|---|
| Gasgenerierendes Grundkörpersystem | 40-60% |
| Tensid(e) | 30-40% |
| haar- und kopfhautpflegewirksame Wirkstoffe | 5-10% |
| Hilfsstoffe und/oder Additive und/oder Stabilisatoren | 1- 5% |

3. Reinigungs- und/oder Pflegemittel nach Anspruch 1 oder 2, dadurch gekennzeichnet, daß dessen Formkörper bzw. Tablette für eine vorwiegende Pflegefunktion als Haarkur, -balsam od.dgl. als Tensid mindestens ein physiologisch unbedenkliches, als Feststoff vorliegendes, kationisches Tensid aus der Gruppe der quartären Ammonium-Verbindungen bzw. -Salze, der Dialkyl- und Tnalkylammonium-Salze, insbesondere -Phosphate oder -Chloride, vorzugsweise Alkyltrimethyl-, Alkyldimethyl- und/oder Alkylmethylammonium-Salze, -Chloride oder-Phosphate enthält.

4. Reinigungs- und/oder Pflegemittel nach Anspruch 3, dadurch gekennzeichnet, daß es als kationisches Tensid zumindest ein Tensid aus der Gruppe der Stearyl-, Lauryl- oder Cetyltrimethylammonium-Chloride oder -Phosphate, Distearyl-, Dilauryl- oder Dicetyldimethylammonium-Chloride oder -Phosphate und der Stearyl-, Lauryl- oder Cetylpyridinium-Chlonde oder -Phosphate enthält.

5. Reinigungs- und/oder Pflegemittel nach einem der Ansprüche 1 bis 4, dadurch gekennzeichnet, daß es als Poren aufweisende(r) Formkörper bzw. Tablette vorliegt.

6. Reinigungs- und/oder Pflegemittel nach einem der Ansprüche 1 bis 5, dadurch gekennzeichnet, daß die Formkörper im Rahmen eines (Farb-) Kennungs- bzw. -Leit-Systems zumindest oberflächen-gefärbt und/oder geprägt sind.

7. Reinigungs- und/oder Pflegemittel nach einem der Ansprüche 1 bis 6, dadurch gekennzeichnet, daß der Grundkörper mit einer die rasche Desintegration des Formkörpers bzw. der Tablette und die Verteilung von deren Inhaltsstoffen durch Entwicklung von CO₂-Gas bei manipulativem Kontakt mit Wasser fördernden Kombination von mindestens einem Alkali-, Pseudoalkali- und/oder Erdalkali-Carbonat und/oder -Hydrogencarbonat, insbesondere Natrium(hydrogen)carbonat oder Magnesium(hydrogen)carbonat, und mindestens einer Säure aus der Gruppe der gesättigten oder ungesättigten, aliphatischen und/oder aromatischen Di- bzw. Polycarbonsäuren, (Poly)hydroxy- (poly)carbonsäuren und (Poly)amino(poly)carbonsäuren gebildet ist.

8. Reinigungs- und/oder Pflegemittel nach einem der Ansprüche 1 bis 8, dadurch gekennzeichnet, daß der desintegrations- und verteilungsfördernde, gasgenerierende Grundkörper des Formkörpers mindestens eine in Festphase vorliegende, physiologisch verträgliche, organische Säure aus der Gruppe Wein-, Zitronen-, Milch-, Glutar-, Fumar-, Bernstein- und Apfelsäure, Alanin, Valin, Leucin, Asparagin, Glutamin, Salicyl-, Sorbin-, Benzoe- und/oder Ascorbinsäure enthält.

9. Reinigungs- und/oder Pflegemittel nach einem der Ansprüche 1 bis 8, dadurch gekennzeichnet, daß dessen Formkörper bzw. Tablette für eine vorwiegende Reinigungs- und Waschfunktion als Tensid mindestens ein anionisches Tensid in fester Phase, aus der Gruppe der C₈-bis C₂₂-, vorzugsweise C₁₂- bis C₂₀- Fettalkohol-Sulfate und/oder Sulfonate, Alkylaryl (benzol)-Sulfate und/oder -Sulfonate, deren Alkali (Na,K)-, Erdalkali (Mg)-, Ammonium-Salze sowie Mono- und Diethanolamide derselben, der üblichen Seifen sowie der C₁₂- bis C₂₀-Fettsäure-Mono- und/oder Diethanolamide, Sulfobernsteinsäureester, Alkylpolyglycolethersulfate oder -sulfonate enthält.

10. Reinigungs- und/oder Pflegemittel nach Anspruch 9, dadurch gekennzeichnet, daß es als anionisches Tensid mindestens ein solches aus der Gruppe (Natrium- oder Magnesium)- Lauryl- oder Cetylsulfat oder -sulfonat, Kokosfettsäuremono- oder -diethanolamid und (Natrium- oder Magnesium)-Cumolsulfat oder -sulfonat enthält.

11. Reinigungs- und/oder Pflegemittel nach einem der Ansprüche 1 bis 10, dadurch gekennzeichnet, daß die es bildenden Formkörper bzw. Tabletten, innerhalb eines eine Vielzahl von Wiederbefüllungen ermöglichenden. an sich bekannten Tabletten-Dispensers od.dgl. angeordnet sind.

12. Verfahren zur Herstellung der neuen Haarreinigungs- und/oder Pflegemittel gemäß einem der Ansprüche 1 bis 11 durch inniges Vermengen der jeweils vorgesehenen Komponenten, gegebenenfalls unter weiterer Zerkleinerung, z.B. durch mahlendes Mischen, Dosieren des erhaltenen Gemenges und Kornpaktieren desselben unter Druckanwendung zu einem jeweils vorgesehenen Formkörper, dadurch gekennzeichnet, daß für den Fall des Einbaues einer in flüssiger Phase vorliegenden Komponente wie insbesondere von ätherischen Ölen, Parfumes oder Propandiol diese flüssige Phase in einer ersten Stufe mit Talkum und/oder der Stärke vermischt und dann in die Mischung mit den restlichen, in fester Form vorliegenden Komponenten eingebracht wird, welche schließlich kompaktiert bzw tablettiert wird.

## Claims

1. Cleansing and care agent for human hair, in the form of solid compacted bodies or tablets based on a solid blend or mixture, the cleansing agent comprising at least one solid surfactant compatible with hair and skin, at least one additive and/or auxiliary agent, and a base compound which, upon contact with water, sets free CO₂ or another physiologically harmless gas, said base compound formed preferably from a combination of at least one carbonate and/or carbamate and/or hydrogen carbonate and at least one organic acid in solid form, characterized in that the cleansing agent is provided in the form of cleansing and care units, each unit having a volume of 0.5 to 2.5 cm³, preferably of 1 to 1.5 cm³, and has the following composition with respect to the total mass of the solid body or tablet:
| | | |
|---|---|---|
| a) | Gas-generating base compound system: | 40 - 60 %, |
| b) | Surfactant(s): | 25 - 45 %, |
| c) | Additive(s) for hair and scalp care: Auxiliary compound(s), additive(s) and | 3 - 12 %, |
| d) | magnesium oxide and/or alkali aluminate as stabilizers: | 1 - 5 %, |
wherein the cleansing agent from the group of substances, mentioned above under c) and d), comprises at least one - generally known - effectual additive for hair and skin scare taken from the groups consisting of
additives and anti-irritants compatible with and beneficial for the skin, preferably allantoin,
radical trapping substances, preferably urea,
avivage softeners, preferably guarhydroxy propyl trimethyl ammonium chloride, growth promoting additives, preferably propandiol,
dandruff additives, preferably octopirox, pyrithion salts, selenium disulfide, anti fat additives, preferably a combination of various (thio) amino acids or isopropyl myristate,
fat retarding additives, preferably amino dermine or proteo-hydrolysates, as well as of
final treatment additives, preferably silk proteins, dried herb extracts and/or distilled oils or their mixtures,
at least one stabilizer taken from the group consisting of magnesium oxide and alkali aluminates and auxiliary agents and at least one additive taken from the groups consisting of
compacting and pressing aids, preferably talcum, fillers, preferably sodium or magnsium sulfates,
burst agents, preferably starch, cellulose derivates, carboxy methyl cellulose, alginates, silicon dioxide or titanium dioxide,
lubricants and gloss agents, preferably cured castor-oil, metal soaps, or the like, dry binders, preferably saccharose, lactose or sorbitol,
as well as
dyes or colorants,
and wherein the relative stoichiometric concentration of the acid in the base compound which promotes disintegration, is higher than the concentration of the components in the base compound which generate the gas, and wherein the cleansing and care agent has a total pH-value of less than 6.8, preferably between 3 and 6, after the solid body or the tablet has disintegrated or dissolved or is distributed in the aqueous phase.

2. Cleansing and/or care agent according to claim 1, characterized in that the cleansing agent has the following composition with respect to the total mass of the solid body or tablet;
| | |
|---|---|
| Gas-generating base compound system: | 40 - 60 %, |
| Surfactant(s): | 30 - 40 %, |
| Additive(s) for hair and scalp care: | 5 - 10 %, |
| Auxiliary compounds and/or additives and/or stabilizers: | 1 - 5 %. |

3. Cleansing and/or care agent according to claim 1 or 2, characterized in that the solid body or tablet, preferably for a care function as hair treatment, balsam or similar, comprises as surfactant at least one physiologically harmless, cationic surfactant in solid form, taken from the group consisting of the quaternary ammonium compounds or salts, of the dialkyl and trialkyl ammonium salts, especially phosphates or chlorides, preferably alkyltrimethyl-, alkyldimethyl- and/or alkylmethyl ammonium salts, chlorides or phosphates.

4. Cleansing and/or care agent according to claim 3, characterized in that it comprises as cationic surfactant at least one surfactant taken from the group consisting of stearyl, lauryl or cetyl trimethyl ammonium chlorides or phosphates, of distearyl, dilauryl or dicetyl dimethyl ammonium chlorides or phosphates, and of stearyl, lauryl or cetyl pyridinium chlorides or phosphates.

5. Cleansing and/or care agent according to any one of claims 1 to 4, characterized in that it is provided as solid body/bodies or tablet and comprises pores.

6. Cleansing and/or care agent according to any one of claims 1 to 5, characterized in that the solid bodies are, within the framework of a (color) coding and control system, colored or embossed at least on the surface.

7. Cleansing and/or care agent according to any one of claims 1 to 6, characterized in that the base compound is formed from a combination of at least one alkali, pseudo-alkali and/or alkaline earth carbonate and/or hydrogen carbonate, especially sodium (hydrogen) carbonate or magnesium (hydrogen) carbonate, and at least one acid from the group of saturated or unsaturated aliphatic and/or aromatic di- or polycarboxylic acids, (poly)hydroxy-(poly)carboxylic acids and (poly)amino(poly)carboxylic acids, wherein said combination promotes the disintegration of the solid body or the tablet and the distribution of its content through the generation of CO₂-gas upon manipulative contact with water.

8. Cleansing and/or care agent according to any one of claims 1 to 7 characterized in that the base compound of the solid body which promotes disintegration and distribution and generates gas, comprises at least one physiologically compatible, organic acid in the solid phase, taken from the group consisting of tartaric acid, citric acid, lactic acid, glutaric acid, fumaric acid, succinic acid and malic acid, alanine, valine, leucine, aspartic acid, glutamic acid, salicylic acid, sorbic acid, benzoic acid and/or ascorbic acid.

9. Cleansing and/or care agent according to any one of claims 1 to 8, characterized in that its solid body or tablet comprises as surfactant for a predominant cleansing and wash function at least one anionic surfactant in the solid phase, taken from the group consisting of the C₈- to C₂₂-, preferably of the C₁₂- to C₂₀-, fatty alcohol sulfates and/or sulfonates, alkyl aryl (benzene) sulfates and/or sulfates, their alkali (Na, K), earth alkaline (Mg), ammonium salts as well as their mono- and di- ethanol amides, of the customary soaps as well as of the C₁₂- to C₂₀- fatty acid mono- and/or diethanolamides, thio-succine acid ester, alkyl polyglycol ether sulfates or sulfonates.

10. Cleansing and/or care agent according to claim 9, characterized in that it comprises as an anionic surfactant at least one surfactant taken from the group consisting of (sodium- or magnesium-) lauryl- or cetyl-sulfate or sulfonate, cocus fatty acid mono- or di-ethanol amide and (sodium- or magnesium-) cumene sulfate or sulfonate.

11. Clansing and/or care agent according to any one of claims 1 to 10, characterized in that the solid bodies or tablets forming said agent are positioned within a known tablet dispenser or similar, which permits a number of refills.

12. Process for producing the new hair cleansing and/or care agents according to any one of claims 1 to 11 through intimate mixing of the selected components, possibly through additional size reduction, for example through grinding and mixing, metering the resulting mixture and compacting the same under applied pressure to obtain the desired solid body, characterized in that, if the addition of a component which exists in liquid phase, like distilled oils, perfumes or propandiol, is desired, this liquid phase is mixed in a first step with talcum and/or the starch and subsequently introduced into the mixture together with the remaining solid components and finally is compacted and pressed into tablets.

## Revendications

1. Produit de lavage et de soin pour cheveux sous forme de corps mis en forme ou de tablettes à base d'un mélange solide comprenant au moins un agent tensio-actif ayant une bonne tolérance pileuse et cutanée et présent en phase solide, au moins un additif et/ou adjuvant et un corps de base libérant, au contact avec de l'eau, du CO₂ ou un autre gaz physiologiquement inoffensif et étant composé de préférence d'une combinaison d'au moins un carbonate et/ou carbaminate et/ou hydrocarbonate et/ou au moins un acide organique présent en phase solide, caractérisé en ce qu'il présente la composition de base suivante, sous forme d'unités ou de sous-unités de lavage et de soin ayant un volume de chaque fois 0,5 à 2,5 cm³, notamment de 1 à 1,5 cm³ rapporté à la masse totale du corps mis en forme ou de la tablette :
| | | |
|---|---|---|
| a) | système de corps de base générant du gaz : | 40 à 60 % |
| b) | agent(s) tensio-actif(s): | 25 à 45 % |
| c) | principes actifs agissant sur les cheveux et le cuir chevelu | 3 à 12 % |
| d) | adjuvant(s) et additif(s) et oxyde de magnésium et/ou aluminate alcalin comme stabilisateurs | 1 à 5 %, |
en comprenant, dans ces deux derniers groupes de substances c) et d), au moins un principe actif de type connu ayant un effet de soin sur les cheveux et sur la peau provenant des groupes
des principes actifs favorisant la tolérance cutanée et des anti-irritants, de préférence de l'allantoïne, capteurs de radicaux, de préférence de l'urée,
principes actifs d'avivage, de préférence du chlorure d'ammonium de guarhydroxypropyltriméthyle,
activateurs de croissance, de préférence du propanediol,
principes actifs anti-pelliculaires, de préférence de l'octopirox, des sels de pyrithione, du bisulfure de sélénium,
principes actifs anti-graisse, de préférence une combinaison de différents acides aminés (thio) ou de l'isopropylmyristal,
inhibiteurs d'accumulation de graisse, de préférence de l'aminodermine ou des produits d'hydrolyse protéique ainsi que des
produits de soin final, de préférence des protéines de soie, des extraits secs d'herbes et/ou des huiles essentielles ou leurs mélanges,
au moins un stabilisateur du groupe oxyde de magnésium et aluminates alcalins et au moins un adjuvant ou additif des groupes des
agents de compactage et adjuvants de compression, de préférence du talc,
des charges, de préférence des sulfates de sodium ou de magnésium,
des agents de fragmentation, de préférence de l'amidon, des dérivés de la cellulose, de la carboxyméthylcellulose, des alginates, des dioxydes de silicium ou des dioxydes de titane,
des lubrifiants et agents de brillance, de préférence de l'huile de ricin durcie, des savons de métal et similaires,
des liants secs, de préférence du saccharose, du lactose ou du sorbitol ainsi que
des teintures ou des colorants,
l'acide étant présent dans le corps de base en désintégration générant du gaz en excédent molaire stoechiométrique par rapport à son composant de développement de gaz, et le produit de lavage et de soin présentant, suite à la désintégration, la diffusion et la solubilisation du corps mis en forme ou de la tablette dans la phase aqueuse, un pH total inférieur à 6,8, notamment de 3-6.

2. Produit de lavage et de soin selon la revendication 1, caractérisé en ce qu'il présente, par rapport à la masse totale du corps mis en forme ou de la tablette, la composition de base suivante :
| | |
|---|---|
| système de corps de base générant du gaz: | 40 à 60 % |
| agent(s) tensio-actif(s): | 30 à 40 % |
| principes actifs agissant sur les cheveux et le cuir chevelu : | 5 à 10 % |
| adjuvant(s) et/ou additif(s) et stabilisateurs | 1 à 5 %. |

3. Produit de lavage et/ou de soin selon l'une quelconque des revendications 1 ou 2, caractérisé en ce que le corps mis en forme ou la tablette contient comme agent tensio-actif, en vue d'une fonction prépondérante de soin en tant que cure pour cheveux, baume pour cheveux et similaires, au moins un tensio-actif cationique physiologiquement inoffensif, présent sous forme solide, du groupe des composés ou sels d'ammonium quaternaires, des sels de dialcoïlammonium et de trialcoïlammonium, notamment phosphates et chlorures, de préférence des sels d'ammonium, chlorures et phosphates d'ammonium alkyltriméthyle, alkyldiméthyle et/ou alkylméthyle.

4. Produit de lavage et/ou de soin selon la revendication 3, caractérisé en ce qu'il contient comme tensio-actif cationique au moins un tensio-actif du groupe des chlorures ou phosphates d'ammonium stéarique, laurique ou cétyltriméthylique, des chlorures ou phosphates d'ammonium distéarique, dilaurique ou dicétyltriméthylique ou des chlorures ou phosphates de pyridinium stéarique, laurique ou cétylique.

5. Produit de lavage et/ou de soin selon l'une quelconque des revendications 1 à 4, caractérisé en ce qu'il existe comme corps mis en forme ou tablette(s) présentant des pores.

6. Produit de lavage et/ou de soin selon l'une quelconque des revendications 1 à 5, caractérisé en ce que les corps mis en forme, dans le cadre d'un système de marquage et de guidage (coloré), sont colorés et/ou marqués au moins à la surface.

7. Produit de lavage et/ou de soin selon l'une quelconque des revendications 1 à 6, caractérisé en ce que le corps de base est composé d'une combinaison d'au moins un carbonate et/ou hydrocarbonate alcalin, pseudoalcalin et/ou alcalino-terreux, notamment du (hydro)carbonate de sodium ou du (hydro)carbonate de magnésium et au moins un acide du groupe des acides dicarboniques ou polycarboniques, des acides (poly)hydroxy(poly)carboniques et des acides (poly)amino(poly)carboniques saturés ou insaturés, aliphatiques et/ou aromatiques, combinaison favorisant la désintégration rapide du corps mis en forme ou de la tablette et la diffusion de leurs ingrédients par dégagement de gaz CO₂ en contact manipulatif avec de l'eau.

8. Produit de lavage et/ou de soin selon l'une quelconque des revendications 1 à 7, caractérisé en ce que le corps de base du corps mis en forme favorisant la désintégration et la diffusion et générant du gaz contient au moins un acide organique physiologique, présent en phase solide, du groupe de l'acide tartrique, citrique, lactique, glutarique, fumarique, succinique et malique, alanine, valine, leucine, asparagine, glutamine, acide sorbique, benzoïque et/ou ascorbique.

9. Produit de lavage et/ou de soin selon l'une quelconque des revendications 1 à 8, caractérisé en ce que le corps mis en forme ou la tablette pour une fonction prépondérante de nettoyage et de lavage contient, comme agent tensio-actif, au moins un tensio-actif anionique en phase solide du groupe C₈ à C₂₂, de préférence C₁₂ à C₂₀- sulfates et/ou sulfonates d'alcool gras, des sulfates et/ou sulfonates d'aryle d'alkyle (benzoïque), leurs sels alcalins (Na, K), alcalino-terreux (Mg), ammonium ainsi que de leurs mono- et diéthanolamides, des savons courants ainsi que des mono- et/ou diéthanolamides des acides gras C₁₂ à C_{20,} des esters d'acide sulfosuccinique, des sulfates ou sulfonates d'éther d'alkylpolyglycol.

10. Produit de lavage et/ou de soin selon la revendication 9, caractérisé en ce qu'il contient, comme tensio-actif anionique, au moins un tensio-actif du groupe des sulfates ou sulfonates laurique ou cétylique (de sodium ou de magnésium), des mono- ou diéthanolamides d'acide gras de coco et des sulfates ou sulfonates de cumène (de sodium ou de magnésium).

11. Produit de lavage et/ou de soin selon l'une quelconque des revendications 1 à 10, caractérisé en ce que les corps mis en forme ou les tablettes le constituant sont disposés à l'intérieur d'un dispenseur de tablettes de type connu ou similaire permettant une pluralité de rechargements.

12. Procédé de fabrication du nouveau produit de lavage et/ou de soin pour cheveux selon l'une quelconque des revendications 1 à 11 par le mélange intime des composants prévus, éventuellement par une plus forte désintégration, par exemple par mélange-broyage, dusage du mélange obtenu et compactage sous pression pour obtenir le corps mis en forme prévu, caractérisé en ce que, en cas de l'intégration d'un composant présent en phase liquide, notamment d'huiles essentielles, de parfums ou de propanediol, cette phase liquide est d'abord mélangée avec du talc et/ou de l'amidon puis introduite dans le mélange contenant les autres composants présents sous forme solide, lequel est finalement compacté ou mis en tablette.
